# EUROPÄISCHE PATENTSCHRIFT

(11) **EP 3 024 409 B1**
(45) Veröffentlichungstag und Bekanntmachung des Hinweises auf die Patenterteilung: **11.01.2023**
(21) Anmeldenummer: 14739420.9
(22) Anmeldetag: 14.07.2014
(51) Int. Cl.: A61B 34/20

(54) **VORRICHTUNG UND VERFAHREN ZUR ANBINDUNG EINES MEDIZINISCHEN INSTRUMENTS AN EIN LAGEERFASSUNGSSYSTEM**
DEVICE AND METHOD FOR CONNECTING A MEDICAL INSTRUMENT TO A POSITION-DETECTING SYSTEM
DISPOSITIF ET PROCÉDÉ DE COUPLAGE D'UN INSTRUMENT CHIRURGICAL À UN SYSTÈME DE DÉTECTION DE POSITION

(30) Priorität: 17.07.2013 DE 102013214067
(43) Veröffentlichungstag der Anmeldung: 01.06.2016
(73) Patentinhaber: Intersect ENT GmbH, 16761 Hennigsdorf (DE)
(72) Erfinder: KRÜGER, Timo, 13469 Berlin (DE); MUCHA, Dirk, 16548 Glienicke (DE)
(74) Vertreter: Eisenführ Speiser
(86) Internationale Anmeldenummer: PCT/EP2014/065030
(87) Internationale Veröffentlichungsnummer: WO 2015/007681

(56) Entgegenhaltungen:
- US-A1- 2004 054 489
- US-A1- 2004 073 279
- US-A1- 2006 161 059
- US-A1- 2006 271 056
- US-A1- 2008 228 195
- US-A1- 2010 210 939
- US-A1- 2010 234 724
- US-A1- 2011 054 449

## Beschreibung

Die Erfindung bezieht sich auf ein Lageerfassungssystem nach Anspruch 1 und ein Verfahren unter Verwendung dieses Lageerfassungssystems nach Anspruch 13.

Das Lageerfassungssystem umfasst eine erste Vorrichtung mit Lokalisierungselement zur Anbindung eines medizinischen Instruments, insbesondere eines medizinischen Instruments für invasive chirurgische Eingriffe, an das Lageerfassungssystem, eine weitere Vorrichtung mit Lokalisierungselement und eine Steuereinheit, wobei das medizinische Instrument einen Instrumentenschaft, eine Instrumentenspitze sowie einen Arbeitspunkt aufweist.

Unter einem Lageerfassungssystem wird hier ein System verstanden, welches Lageinformation zu einem medizinischen Instrument liefert. Die Lageinformation ist nützlich, um ein Instrument im Zusammenhang mit Bildgebungseinrichtungen wie Topographen oder tomographisch gewonnenen Bilddaten einzusetzen. Unter Lageinformation werden hier die Position und die Ausrichtung des medizinischen Instruments in Bezug auf ein Referenzkoordinatensystem verstanden. Ein Referenzkoordinatensystem ist ein Koordinatensystem einer Lageerfassungseinrichtung bzw. eines Lageerfassungssystems. Die Lageinformation kann mittels einer Lageerfassungseinrichtung mit einem entsprechenden Messsystem erfasst werden.

Bei der Durchführung invasiver chirurgischer Eingriffe werden als Planungsdaten oft präoperativ gewonnene Bilddaten des Patienten, z.B. Computertomographieaufnahmen verwendet. Darüber hinaus ist es üblich, Bilder des Patienten intraoperativ, z.B. mit einem Endoskop aufzunehmen und zusammen mit den präoperativ erstellten Bilddaten auf einem Monitor jeweils als Einzelbild oder überlagert darzustellen. Auf diese Weise können beispielsweise zu entfernendes Gewebe oder im Operationsgebiet liegende und durch den chirurgischen Eingriff potenziell gefährdete Nervenbahnen oder Gefäße dem Chirurgen besser sichtbar gemacht werden. Diese Darstellung der Bilddaten kann dem Chirurgen somit helfen, die bei der Operation verwendeten medizinischen Geräte möglichst effizient und unter minimaler Beeinträchtigung des umliegenden Gewebes des Patienten einzusetzen. Dabei muss der Chirurg zunächst das medizinische Instrument möglichst genau entlang einem bestimmten Weg in ein meist präoperativ festgelegtes Eingriffsgebiet im Körper des Patienten navigieren und in dem Eingriffsgebiet die operative Maßnahme, z.B. das Entfernen von Gewebeteilen, möglichst präzise durchführen. Im Anschluss an die operative Maßnahme muss das medizinische Instrument wieder kontrolliert aus dem Körper des Patienten heraus navigiert werden. Demnach erfordern invasive chirurgische Eingriffe oftmals eine hohe Genauigkeit der ermittelten Lageinformationen von medizinischen Instrumenten.

Ferner kann die gedankliche Verknüpfung zwischen den vorhandenen Bilddaten und der Steuerung der medizinischen Instrumente leicht zu Orientierungsproblemen des Chirurgen und somit zu Operationsfehlern und/oder zu einer Verlängerung der Operation führen.

Aus diesem Grund kommen als Orientierungshilfe für den Chirurgen während der Operation regelmäßig Positionserfassungs- bzw. Lageerfassungssysteme zum Einsatz, um beispielsweise eine Navigation von medizinischen Instrumenten, insbesondere chirurgischen Instrumenten, zu unterstützen. Derartige Systeme erfassen während der Operation die Koordinatentransformation zwischen dem Patienten und mindestens einem medizinischen Instrument. In vielen Lageerfassungssystemen können Lageinformationen einer Vielzahl von unterschiedlichen medizinischen Instrumenten ermittelt werden. Die gewonnenen Lageinformationen werden i.d.R. zusammen mit den präoperativen Planungsdaten und/oder den intraoperativen Bilddaten auf einem Monitor visualisiert. Hierfür werden an bestimmten Stellen des Patienten sowie an den medizinischen Instrumenten Lokalisierungselemente angeordnet, deren Lageinformationen von einem Messsystem als Lageerfassungseinrichtung erfasst werden. Auf diese Weise sind zunächst die Lageinformationen der Lokalisierungselemente im Lageerfassungssystem ermittelbar.

Derartige Lageerfassungssysteme können beispielsweise optische, ultraschallgestützte oder elektromagnetische Lokalisierungselemente umfassen. Bekannt sind beispielsweise elektromagnetische Lageerfassungssysteme, die einen Feldgenerator aufweisen. Der Feldgenerator erzeugt in einem Operationsgebiet ein i.d.R. alternierendes elektrisches Wechselfeld. An einem in diesem Operationsgebiet zu navigierenden medizinischen Instrument sind Lokalisierungselemente angeordnet, die Spulen aufweisen. Das elektromagnetische Wechselfeld induziert in diesen Spulen in Abhängigkeit von Ausrichtung der jeweiligen Spule zum elektromagnetischen Wechselfeld charakteristische Ströme, aus denen die Lageinformationen der Spulen bestimmbar sind.

Des Weiteren ist bekannt, unterschiedliche medizinische Instrumente, wie z.B. Zeigeinstrument, Sauger, Zange, Nadeln, Skalpell, Elektrotom, Kauter, u.ä. mit Lokalisierungselementen zur Ermittlung der Lageinformationen für ein solches Lageerfassungssystem zu versehen und das jeweilige medizinische Instrument in dem Lageerfassungssystem zu registrieren. Bei der Registrierung des medizinischen Instruments wird die Lage eines Bezugspunkts - üblicherweise des Arbeitspunkts der Instrumentenspitze - relativ zum an dem medizinischen Instrument angeordneten Lokalisierungselement eingemessen und an das Lageerfassungssystem übermittelt. Somit sind die Lage des Bezugspunkts und Ausrichtung des medizinischen Instruments im Koordinatensystem des Lageerfassungssystems bekannt und können auf dem Monitor zusammen mit den vorhandenen Bilddaten dargestellt werden.

Eine mit einem Instrument zusammenwirkende Vorrichtung zum Tracking ist aus der US 2010/0234724 A1 bekannt. Ein Gerät zum Kalibrieren eines chirurgischen Werkzeugs mit einem elektronischen Sensormodul ist in der US 2011/0054449 A1 beschrieben. US 2004/0054489 A1 beschreibt ein Verfahren zum Kalibrieren der Spitzenposition eines chirurgischen Instruments sowie dessen Orientierung. In diesem Dokument wird eine Werkzeugspitze eines chirurgischen Werkzeugs mit einer universellen Tracking-Vorrichtung gegen einen Kalibrierungspunkt einer Referenz-Tracking-Vorrichtung platziert. Sowohl die Lage des Kalibrierungspunkts auf der Referenz-Tracking-Vorrichtung als auch die Position der universellen Tracking-Vorrichtung sind einem chirurgischen Navigationssystem bekannt. Aufgrund dieser bekannten Beziehung kann das chirurgische Navigationssystem die Position der Werkzeugspitze relativ zur universellen Tracking-Vorrichtung bestimmen, wenn die Werkzeugspitze gegen den Kalibrierungspunkt gehalten wird. Ein Tracking Array zum Befestigen an einem Objekt in einer chirurgischen Umgebung, wobei das Array Tracking-Elemente aufweist, ist aus der US 2006/0161059 A1 bekannt. Ein Instrument zur Verwendung in einer computerunterstützten Operation wird in der US 2006/0271056 A1 beschrieben. US 2010/0210939 A1 offenbart ein chirurgisches Navigationssystem zum Tracken eines Instruments relativ zu einem Patient. Ein Instrumentenleitsystem mit einer Griffanordnung und einer Instrumentenbefestigungsanordnung ist aus der US 2008/0228195 A1 bekannt.

Voraussetzung für eine Registrierung eines medizinischen Instruments in einem Lageerfassungssystem ist, dass das medizinische Instrument wenigstens ein Lokalisierungselement aufweist. Allerdings gibt es eine Vielzahl von medizinischen Instrumenten, die über kein derartiges Lokalisierungselement verfügen und somit in einem Lageerfassungssystem nicht einsetzbar sind.

Demnach ist es die Aufgabe der vorliegenden Erfindung, eine Vorrichtung zur Anbindung eines medizinischen Instruments an ein Lageerfassungssystem bereitzustellen, so dass medizinische Instrumente, die über kein eigenes Lokalisierungselement verfügen, in einem Lageerfassungssystem registrierbar sind, damit Lageinformationen des jeweiligen medizinischen Instruments vom Lageerfassungssystem bestimmbar und z.B. auf einem Monitor darstellbar sind.

Erfindungsgemäß wird die Aufgabe durch eine Vorrichtung mit einem Lokalisierungselement zur Anbindung eines medizinischen Instruments an einem Lageerfassungssystem gelöst, wobei das medizinische Instrument einen Instrumentenschaft, eine Instrumentenspitze sowie einen Arbeitspunkt aufweist. An der Vorrichtung ist wenigstens ein Lokalisierungselement zum Erfassen von Lageinformationen, wie etwa Ausrichtung und Position, der Vorrichtung relativ zu einem Bezugspunkt anordenbar. Des Weiteren weist die Vorrichtung ein Haltemittel zur Befestigung an dem medizinischen Instrument auf.

Vorzugsweise weist das Haltemittel eine Instrumentenaufnahme auf, wobei zumindest ein Teilabschnitt des medizinischen Instruments mit der Instrumentenaufnahme in Eingriff zu bringen und über mindestens ein Fixiermittel an der Instrumentenaufnahme zu befestigen ist. Die Instrumentenaufnahme kann beispielsweise schalen- oder zylinderförmig ausgebildet sein. Vorzugsweise ist die Instrumentenaufnahme ausgebildet, einen Teilbereich des medizinischen Instruments zu umfassen. Weiter bevorzugt ist die Instrumentenaufnahme an dem instrumentenspitzenseitigen, also dem distalen Ende des medizinischen Instruments auf das medizinische Instrument aufsetzbar und entlang einer Instrumentenlängsachse auf dem Instrument verschiebbar. Vorzugsweise ist die Instrumentenaufnahme am Instrumentenschaft über das Fixiermittel zu befestigen.

Bevorzugt umfasst das Fixiermittel eine Schraube. Die Schraube ist beispielsweise so in die Vorrichtung schraubbar, dass im Ergebnis ihre vorzugsweise abgeflachte Schraubenspitze gegen einen von der Instrumentenaufnahme umfassten Abschnitt des Instrumentenschafts presst, um somit das medizinische Instrument in der Instrumentenaufnahme zu halten.

In einer bevorzugten Ausführungsform der Erfindung weist das Fixiermittel eine Klemmeinrichtung auf, um das medizinische Instrument in der Instrumentenaufnahme zu halten. Die Klemmeinrichtung kann beispielsweise Klemmbacken aufweisen, die über einen Schraubmechanismus mit dem medizinischen Instrument zu verspannen sind.

Vorzugsweise weist die Vorrichtung eine Sensoraufnahme auf, die das Lokalisierungselement aufnehmen kann. Hierbei sind sowohl Ausführungsformen vorgesehen, bei denen das Lokalisierungselement reversibel als auch Ausführungsformen, bei denen das Lokalisierungselement irreversibel in die Sensoraufnahme aufzunehmen ist.

In einer bevorzugten Ausführungsform der Erfindung weisen die Sensoraufnahme und das Lokalisierungselement Führungsmittel auf, über die Sensoraufnahme und Lokalisierungselement miteinander derart zu verbinden oder in Eingriff bringbar sind, dass Sensoraufnahme und Lokalisierungselement relativ zueinander auf einer Führungsbahn verschiebbar sind. Hierbei kann beispielsweise die Sensoraufnahme einen schienenförmigen Teil und das Lokalisierungselement einen entsprechenden schlittenförmigen Teil aufweisen. Alternativ kann die Sensoraufnahme einen schlittenförmigen Teil und das Lokalisierungselement einen schienenförmigen Teil aufweisen.

Vorzugsweise sind die Führungsmittel der Sensoraufnahme und/oder des Lokalisierungselements derart ausgebildet, dass ein relatives Verfahren von Sensoraufnahme und Lokalisierungselement nur bis zu einem definierten Punkt auf der Führungsbahn möglich ist. Hierfür kann mindestens ein Teil der Führungsbahn zum Beispiel einen Zahn oder einen Absatz aufweisen.

Vorzugsweise weisen die Führungsmittel von Sensoraufnahme und/oder Lokalisierungselement ein Rückhaltemittel mit einem Federelement auf, wobei das Lokalisierungselement durch das Rückhaltemittel in einer vordefinierten Position auf der Führungsbahn fixierbar ist. Das Rückhaltemittel ist vorzugsweise ausgebildet, durch das Verfahren des Lokalisierungselements entlang der Führungsbahn die Fixierung auszulösen. Hierfür kann der schienenförmige Teil des Führungsmittels beispielsweise eine Nut und der schlittenförmige Teil des Führungsmittels einen federnd gehaltenen Keil aufweisen, der durch die Federkraft in die Nut des schienenförmigen Teils schwenken kann und somit die Fixierung bewirkt.

In einer bevorzugten Ausführungsform der Erfindung ist das Lokalisierungselement an dem Haltemittel fest angeordnet. Eine Demontage des Lokalisierungselements von dem Haltemittel ist in einer derartigen Ausführungsform nicht vorgesehen.

Vorzugsweise weist die Vorrichtung eine Außenfläche mit im Wesentlichen zylinderförmiger Ausprägung auf. Die Außenfläche kann aber auch im Wesentlichen quaderförmig bzw. vieleckförmig ausgebildet sein.

Erfindungsgemäß ist die Vorrichtung ausgebildet, über ein Kabel mit einer Steuereinheit des Lageerfassungssystems verbunden zu werden, um Signale von dem Lokalisierungselement an die Steuereinheit des Lageerfassungssystems zu übermitteln. Hierfür kann die Vorrichtung ein Kabel oder einen Anschluss für ein Kabel, wie z.B. eine Steckbuchse, aufweisen. In einem Beispiel, das nicht Bestandteil der Erfindung ist, kann die Vorrichtung die Signale des Lokalisierungselements über eine Funkeinrichtung an die Steuereinheit des Lageerfassungssystems senden. In einer derartigen kabellosen Ausführungsform weist die Vorrichtung bevorzugt Mittel zur Bereitstellung eines Versorgungsstroms, wie beispielsweise einen Akkumulator oder eine Batterie, auf.

Bevorzugt weist das Haltemittel einen sich in Längsrichtung des Haltemittels erstreckenden Kanal auf. Der Kanal ist zu beiden Stirnseiten des Haltemittels hin offen, so dass zumindest die Instrumentenspitze des medizinischen Instruments durch den Kanal durchführbar ist.

Weiter bevorzugt weist der Kanal einen im Wesentlichen ovalen Querschnitt auf. Der Vorteil des ovalen Querschnitts besteht insbesondere darin, dass das Haltemittel entlang einem gekrümmten Bereich einer Instrumentenlängsachse einer biegesteifen sowie gekrümmten Instrumentenspitze verschiebbar ist.

Erfindungsgemäß ist ein mit der Vorrichtung an dem medizinischen Instrument gehaltenes Lokalisierungselement auf den Arbeitspunkt des medizinischen Instruments kalibrierbar. Dies geschieht über ein Verfahren bei dem mit dem Arbeitspunkt der Instrumentenspitze ein Referenzpunkt angefahren und gleichzeitig die Lage sowie Orientierung des an dem Instrument gehaltenen Lokalisierungselements erfasst wird. Bei Erreichen des Referenzpunkts wird somit das Lokalisierungselement auf die Instrumentenspitze kalibriert. Vorzugsweise wird in dem Moment, in dem der Arbeitspunkt der Instrumentenspitze den Referenzpunkt erreicht, der Arbeitspunkt automatisch kalibriert. Dies wird z.B. durch einen Drucksensor auf dem Referenzpunkt oder einen elektrischen Stromkreis, der durch den Kontakt vom Arbeitspunkt der Instrumentenspitze und Referenzpunkt geschlossen wird, ermöglicht. Gemäß der Erfindung sieht das Verfahren vor, dass als Startbedingung für eine Kalibrierung des Lokalisierungselements auf die Instrumentenspitze geprüft wird, ob sich der anzufahrende Referenzpunkt innerhalb eines Suchsektors befindet, der auf das an dem medizinischen Instrument gehaltene/zu haltende Lokalisierungselement bezogen ist. Der Suchsektor kann je nach Instrument unterschiedlich vorgebbar sein. Bevorzugt ist der Suchsektor kegelförmig ausgebildet. Die Kegelachse liegt insbesondere koaxial zu einer Längsachse der Instrumentenspitze und/oder koaxial zu einem zum Durchführen des Instruments bestimmten Kanal des Haltemittels. Die Kegelspitze zeigt vorzugsweise zum und/oder liegt bevorzugt im Zentrum Lokalisierungselements. Vorzugsweise ist der Suchsektor derart vorgegeben, dass sich eine Instrumentenspitze innerhalb des Suchsektors befindet, wenn das Lokalisierungselement am medizinischen Instrument gehalten ist.

Alternativ (oder zusätzlich) kann die Vorrichtung auch so ausgebildet sein, dass eine automatische Kalibrierung dann erfolgt, wenn der Arbeitspunkt (d.h. z.B. die Instrumentenspitze) für eine vordefinierte Zeit auf dem Referenzpunkt verweilt. Nach erfolgreicher Kalibrierung sind die Lagedaten des Arbeitspunkts der Instrumentenspitze über die vom jeweiligen Lokalisierungselement bereitgestellten Lageinformationen vom Lageerfassungssystem bestimmbar.

Besonders bevorzugt ist der Referenzpunkt an einem an dem Lageerfassungssystem angebundenen sowie registrierten Lokalisierungselement angeordnet. Das Lokalisierungselement kann ebenfalls das Lokalisierungselement eines Instruments sein, so dass zwei medizinische Instrumente im Lageerfassungssystem leicht gegenseitig registrierbar sind. Somit ist die genaue Position des Referenzpunkts innerhalb des Lageerfassungssystems leicht bestimmbar. Der auf dem Lokalisierungselement angeordnete Referenzpunkt ist für ein Einmessen eines neuen mit einem weiteren Lokalisierungselement ausgestatteten Instruments auf den Arbeitspunkt des Instruments geeignet. Ein solcher Kalibriervorgang kann demnach auch intraoperativ erfolgen. Ein weiterer oder alternativer Referenzpunkt kann ein Lokalisierungselement sein, das am Patienten angeordnet und zur Bestimmung der Lageinformationen des Patienten innerhalb des Lageerfassungssystems eingerichtet ist.

Es ist besonders bevorzugt, wenn der Referenzpunkt als Mulde oder als Erhebung (z.B. an einem zweiten Instrument) ausgebildet ist, um eine Selbstzentrierung eines entsprechenden medizinischen Instruments am Referenzpunkt zu ermöglichen. Ein muldenförmiger Referenzpunkt ist besonders gut für das Selbstzentrieren eines Instruments mit einer spitzenförmigen Instrumentenspitze mit dem Referenzpunkt geeignet, da die Instrumentenspitze in die Mulde einführbar ist und somit entlang einer Innenfläche der Mulde zum Mittelpunkt dieser geführt wird. Weiter bevorzugt weist der Mittelpunkt der Mulde eine weitere lokale Vertiefung mit kleinerem Muldenradius auf, in die eine Instrumentenspitze einführbar ist. Somit wird das Anfahren des Muldenmittelpunkts mit der Instrumentenspitze weiter erleichtert. Ein als Erhebung ausgebildeter Referenzpunkt ist besonders für das Kalibrieren von Instrumenten geeignet, die eine rohrförmige sowie einen inneren Kanal aufweisende Instrumentenspitze umfassen, wie z.B. Sauger.

Gemäß der Erfindung weist das Lokalisierungselement eine Sensorspule auf. Sensorspulen sind geeignet für den Einsatz in Positionserfassungssystemen mit einem von einem Feldgenerator erzeugten elektromagnetischen Wechselfeld.

Gemäß der Erfindung ist das Lageerfassungssystem eingerichtet, mehrere Lokalisierungselemente zu registrieren und gleichzeitig zu verwalten. Somit können die Lageinformationen mehrerer mit Lokalisierungselementen ausgestatteter medizinischer Instrumente gleichzeitig ermittelt sowie grafisch und/oder numerisch dargestellt werden. Das Verwalten mehrerer Lokalisierungselemente kann beispielsweise über Multiplexing erfolgen. Beim Multiplexing werden die registrierten Lokalisierungselemente in einem vorgegebenen Takt angesteuert, so dass zu einem Zeitpunkt jeweils nur ein bestimmtes Lokalisierungselement vom Lageerfassungssystem angesteuert wird. Hierbei ist es vorteilhaft, wenn das Lageerfassungssystem zwischen aktiven und inaktiven Instrumenten unterscheiden kann. Inaktive Instrumente können somit vom Lageerfassungssystem, z.B. über einen entsprechenden Filter, ausgeblendet oder die entsprechenden Lokalisierungselemente deaktiviert werden, um die Stabilität der Signale der Lokalisierungselemente der aktiven Instrumente zu erhöhen.

Hierfür kann der Multiplexer z.B. so konfiguriert sein, dass zunächst alle Lokalisierungselemente aktiviert sind und in dem vorgegebenen Takt angesteuert werden. Sobald ein Lokalisierungselement eines in einem Operationsgebiet angeordneten Instruments detektiert wird, werden die übrigen Lokalisierungselemente deaktiviert oder die entsprechenden Instrumente ausgeblendet bis das aktive Instrument das Operationsgebiet wieder verlässt. Für die Verweildauer des aktiven Instruments im Operationsgebiet wird das entsprechende Lokalisierungselement vorzugsweise kontinuierlich vom Lageerfassungssystem erfasst. Das Lageerfassungssystem kann bevorzugt manuell konfiguriert werden, eine größere Anzahl als ein Instrument, z.B. zwei, gleichzeitig im Operationsgebiet über Multiplexing zu erfassen und die übrigen Lokalisierungselemente erst zu deaktivieren oder die entsprechenden Instrumente auszublenden, wenn diese vorgegebene Anzahl an im Operationsgebiet angeordneten, aktivierten Instrumenten erreicht ist.

In einer alternativen Ausgestaltung der Erfindung kann eine Vorrichtung vorgesehen sein, um ein bestimmtes Instrument manuell einzublenden und/oder das jeweilige Lokalisierungselement zu aktivieren. Dies kann z.B. über einen am Instrumentengriff angeordneten Drucksensor erfolgen, der automatisch erkennt, wenn das Instrument in die Hand genommen wird. Die Steuerung kann derart konfiguriert sein, dass das entsprechende Instrument solange aktiv und eingeblendet bleibt, bis ein anderes Instrument aktiviert bzw. eingeblendet wird. Das System kann eingerichtet sein, mehrere aktive Instrumente gleichzeitig zu verwalten. Ferner kann das System eingerichtet sein, ein in dem Operationsgebiet angeordnetes, aktiviertes Instrument nicht automatisch zu deaktivieren oder auszublenden. Somit ist sichergestellt, dass ein Instrument z.B. mindestens solange aktiv bleibt, wie es innerhalb des Operationsgebiets angeordnet ist.

Alternativ kann die Instrumentenhalterung z.B. eingerichtet sein, inaktive Instrumente, die auf einer Instrumentenhalterung abgelegt sind, zu erkennen und diese Information an das Lageerfassungssystem weiterzuleiten, damit das Lageerfassungssystem diese inaktiven Instrumente deaktiviert oder ausblendet.

Die Erfindung soll nun anhand eines Ausführungsbeispiels mit Bezug auf die Figuren näher erläutert werden. In den Figuren zeigt
- Fig. 1: eine schematische Seitenansicht eines medizinischen Instruments mit daran angebrachtem Lokalisierungselement;
- Fig. 2: die Ansicht des medizinischen Instruments aus Fig. 1, wobei an der Instrumentenspitze ein weiteres Lokalisierungselement angeordnet ist; und
- Fig. 3: eine frontale Ansicht einer Steuereinheit mit daran gekoppelten Lokalisierungselementen, wobei ein Lokalisierungselement auf einem medizinischen Instrument angeordnet ist.

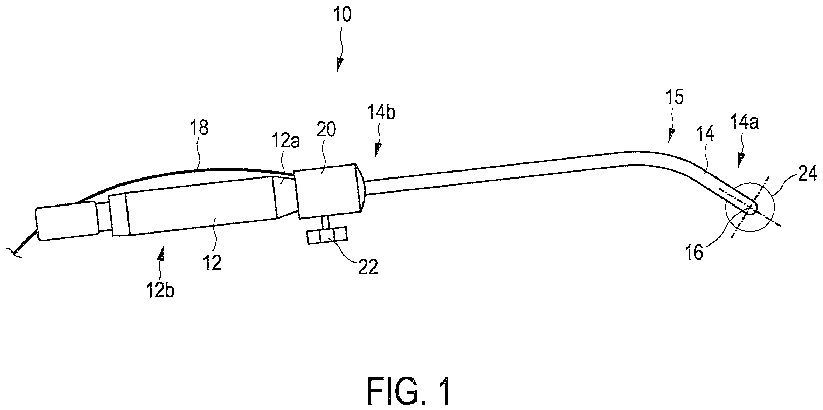

Das in Fig. 1 dargestellte medizinische Instrument 10 weist einen Instrumentenschaft 12 und eine Instrumentenspitze 14 auf. Der Instrumentenschaft 12 weist einen distalen Schaftbereich 12a und einen Griffbereich 12b auf, wobei ein proximaler Spitzenbereich 14b der Instrumentenspitze 14 an dem distalen Schaftbereich 12a angeordnet ist. Der Instrumentenschaft 12 kann mit der Instrumentenspitze 14 einteilig oder mehrteilig, voneinander trennbar ausgebildet sein.

Die Instrumentenspitze 14 weist einen distalen Spitzenbereich 14a auf, an dessen dem proximalen Spitzenbereich 14b abgewandten Ende ein Arbeitspunkt 16 angeordnet ist. Des Weiteren weist die Instrumentenspitze 14 einen gekrümmten Bereich 15 auf, der dem distalen Spitzenbereich 14a benachbart ist. In alternativen Ausführungsformen kann die Instrumentenspitze 14 auch stärkere Krümmungen als abgebildet bzw. weitere Krümmungen aufweisen. Instrumentenschaft 12 und Instrumentenspitze 14 weisen einen im Wesentlichen runden Querschnitt auf.

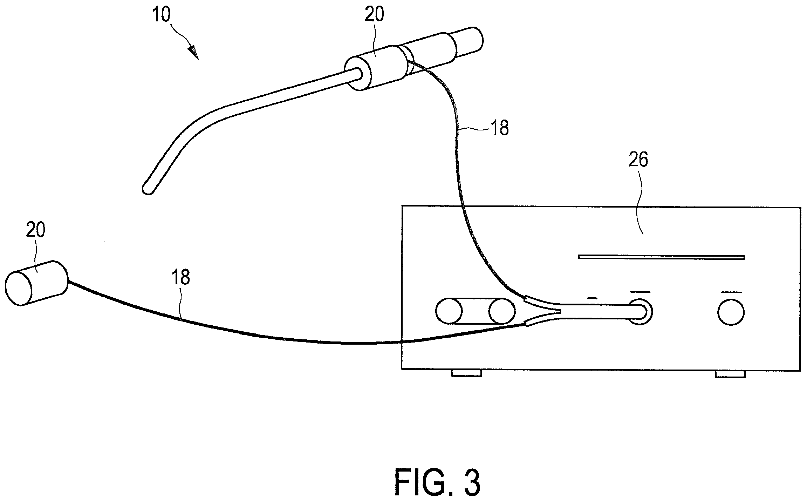

An dem proximalen Spitzenbereich 14b der Instrumentenspitze 14 ist ein Lokalisierungselement 20 dem distalen Schaftbereich 12a benachbart angeordnet. Gemäß der abgebildeten Ausführungsform weist der distale Schaftbereich 12a eine in Richtung Instrumentenspitze 14 verlaufende konische Verjüngung auf, die zumindest teilweise mit dem Lokalisierungselement 20 in Eingriff zu bringen ist. Das Lokalisierungselement 20 ist über eine Klemmschraube 22 an dem proximalen Spitzenbereich der Instrumentenspitze gegen ein Verschieben entlang einer Längsachse der Instrumentenspitze 14 gesichert. Ferner weist Lokalisierungselement 20 ein Kabel 18 zur Verbindung mit einer in Fig. 3 abgebildeten Steuereinheit 26 auf.

Über einen Referenzpunkt 24 ist das Lokalisierungselement 20 auf den Arbeitspunkt 16 des medizinischen Instruments 10 zur Kalibrierung einmessbar. Der Einmessvorgang kann im Wesentlichen automatisch erfolgen, indem der Operateur den Arbeitspunkt 16 des medizinischen Instruments 10 mit dem Referenzpunkt 24 in Kontakt bringt und z.B. eine vordefinierte Zeit - z.B. 1 bis 2 Sekunden - in diesem Zustand verweilt. Alternativ kann z.B. der Referenzpunkt 24 einen hier nicht dargestellten Sensor aufweisen, der den Moment des Kontaktierens von dem Arbeitspunkt 16 mit dem Referenzpunkt 24 erfasst und ein entsprechendes Signal an die Steuereinheit 26 weiterleitet, damit die Steuereinheit 26 die Lagedaten des Lokalisierungselements 20 im Zeitpunkt des Kontaktierens von Arbeitspunkt 16 mit Referenzpunkt 24 erfassen und den Einmessvorgang somit abschließen kann. Nach erfolgtem Einmessen können die Lageinformationen des Arbeitspunkts 16 des medizinischen Instruments in einem Operationsgebiet über die von Lokalisierungselement 20 übermittelten Sensorsignale an das Lageerfassungssystem bestimmt werden.

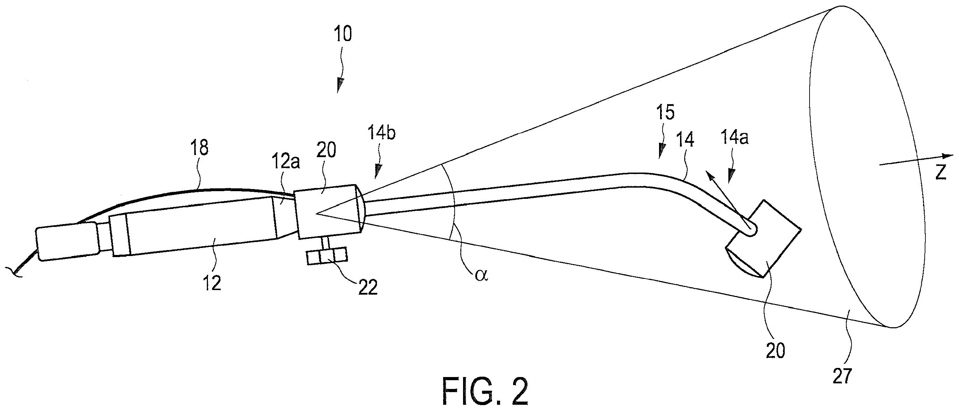

Wie aus Fig. 2 hervorgeht, lassen sich an einem medizinischen Instrument 10 erfindungsgemäß mehrere Lokalisierungselement 20 anordnen. Ein Lokalisierungselement 20 ist in dieser Ansicht bereits wie schon in Fig. 1 auf dem proximalen Spitzenbereich 14b der Instrumentenspitze 14 angeordnet, während ein weiteres Lokalisierungselement 20 noch am distalen Spitzenbereich 14a gehalten ist und zum proximalen Spitzenbereich 14b entlang der Spitzenlängsachse verschiebbar ist. Der Vorteil der Verwendung mehrerer Lokalisierungselemente 20 an einem medizinischen Instrument 10 besteht darin, dass die Lokalisierungselemente 20 sich ergänzende und zum Teil redundante Lageinformationen bereitstellen und somit Messschwankungen bzw. Messfehler von der Steuereinheit 26 des Lageerfassungssystems erkennbar sind.

Ebenfalls in Fig. 2 gezeigt ist ein kegelförmig ausgebildeter Suchsektor 27. Der Suchsektor 27 ist auf das an dem medizinischen Instrument 10 im proximalen Spitzenbereich 14b gehaltene Lokalisierungselement 20 bezogen. Die Kegelachse z liegt koaxial zu der Längsachse der Instrumentenspitze 14. Die Kegelspitze des kegelförmig ausgebildeten Suchsektors 27 zeigt zum und liegt im Zentrum des Lokalisierungselements 20, das im proximalen Spitzenbereich 14b gehalten ist. Der kegelförmige Suchsektor 27 geht somit von dem Lokalisierungselement 20 aus und erstreckt und erweitert sich in distale Richtung. Vorzugsweise endet er in einem definierten Abstand vom Lokalisierungselement 20 und definiert somit einen allseits begrenzten Suchraum. Der Suchsektor 27 ist vorliegend derart vorgegeben, dass sich eine Instrumentenspitze 14 innerhalb des Suchsektors 27 befindet, wenn das Lokalisierungselement 20 am medizinischen Instrument 10 gehalten ist. Der Suchsektor 27 kann auch eine andere Form haben und muss nicht kegelförmig sein. Zweck des Suchsektors 27 ist es, eine automatische Kalibrierung nur dann auszulösen, wenn sich ergibt, dass die von dem Lageerfassungssystem erfassten Positionsdaten des Referenzpunktes 24 innerhalb des von dem Suchsektor 27 aufgespannten Positionsdatenraums (Menge der in den Suchsektor fallenden Positionsdaten) befinden. Bei Ausführungsformen des medizinischen Instruments 10 mit voneinander trennbaren Instrumentenschaft 12 und Instrumentenspitze 14 kann das Lokalisierungselement 20 ausgebildet sein, direkt auf den proximalen Spitzenbereich 14b aufgesetzt zu werden, wenn Instrumentenschaft 12 und Instrumentenspitze 14 voneinander getrennt sind. Nach dem Aufsetzen des Lokalisierungselements 20 können Instrumentenschaft 12 und Instrumentenspitze 14 wieder miteinander verbunden werden.

Alternativ sind Ausführungsformen vorgesehen, bei denen das Lokalisierungselement 20 auf den Instrumentenschaft 12 aufgesetzt werden kann. Hierfür sind prinzipiell die gleichen Haltemittel wie beim Befestigen an der Instrumentenspitze 14 geeignet, wobei die Dimensionen des Instrumentenschafts 12 zu berücksichtigen sind.

Erfindungsgemäß wird eine Steuereinheit 26 (wie in Fig. 3 dargestellt) mit mehreren Lokalisierungselementen 20 über vorzugsweise jeweils ein Kabel 18 verbunden.

Dabei sind an einem medizinischen Instrument 10 jeweils ein oder mehrere Lokalisierungselemente 20 anordenbar. Ferner können die Lokalisierungselemente 20 an unterschiedlichen medizinischen Instrumenten 10 angeordnet werden, wobei in Fig. 3 nur ein medizinisches Instrument 10 abgebildet ist. Die Steuereinheit 26 kann einen Filter aufweisen, um inaktive Instrumente 10, die beispielsweise an einer nicht dargestellten Instrumentenhalterung angeordnet sind, auszublenden. Hierdurch wird die Stabilität der Signale der aktiven Instrumente 10 erhöht.

### Bezugszeichenliste:

- 10: Medizinisches Instrument
- 12: Instrumentenschaft
- 12a: Distaler Schaftbereich
- 12b: Griffbereich
- 14: Instrumentenspitze
- 14a: Distaler Spitzenbereich
- 14b: Proximaler Spitzenbereich
- 15: Gekrümmter Bereich
- 16: Arbeitspunkt
- 18: Kabel
- 20: Lokalisierungselement
- 22: Klemmschraube
- 24: Referenzpunkt
- 26: Steuereinheit
- 27: Suchsektor
- z: Kegelachse

## Patentansprüche

1. Lageerfassungssystem umfassend eine erste Vorrichtung mit Lokalisierungselement (20) zur Anbindung eines medizinischen Instruments (10) an das Lageerfassungssystem, eine weitere Vorrichtung mit Lokalisierungselement und eine Steuereinheit (26),
wobei das medizinische Instrument (10) einen Instrumentenschaft (12), eine Instrumentenspitze (14) sowie einen Arbeitspunkt (16) aufweist,
wobei jedes Lokalisierungselement (20) zur Ermittlung von Lageinformationen der jeweiligen Vorrichtung innerhalb eines Koordinatensystems des Lageerfassungssystems an der jeweiligen Vorrichtung angeordnet ist,
wobei die erste Vorrichtung mindestens ein Haltemittel zur Befestigung an dem medizinischen Instrument (10) aufweist
und jedes Lokalisierungselement (20) eine Sensorspule für den Einsatz in Positionserfassungssystemen mit einem von einem Feldgenerator erzeugten elektromagnetischen Wechselfeld aufweist
und jede Vorrichtung über ein jeweiliges Kabel (18) mit der Steuereinheit (26) des Lageerfassungssystems verbindbar ist, um Signale von den Lokalisierungselementen (20) an die Steuereinheit (26) zu übermitteln,
wobei, die weitere Vorrichtung auf dem Lokalisierungselement einen Referenzpunkt aufweist,
und die Steuereinheit (26) ausgebildet ist, das Lokalisierungselement der ersten Vorrichtung über den Referenzpunkt auf den Arbeitspunkt des medizinischen Instruments zur Kalibrierung einzumessen und
als Startbedingung für eine Kalibrierung des Lokalisierungselements der ersten Vorrichtung auf die Instrumentenspitze zu prüfen, ob sich der Referenzpunkt innerhalb eines von einem Suchsektor aufgespannten Positionsdatenraums befindet, der auf das Lokalisierungselement der ersten Vorrichtung bezogen ist.

2. Lageerfassungssystem nach Anspruch 1,
**dadurch gekennzeichnet, dass**
das Haltemittel eine Instrumentenaufnahme aufweist, wobei zumindest ein Teilabschnitt des medizinischen Instruments (10) mit der Instrumentenaufnahme in Eingriff bringbar und über mindestens ein Fixiermittel der ersten Vorrichtung an der Instrumentenaufnahme befestigbar ist.

3. Lageerfassungssystem nach Anspruch 1 oder 2,
**dadurch gekennzeichnet, dass**
das Fixiermittel eine Schraube umfasst, die so in die erste Vorrichtung schraubbar ist, dass im Ergebnis ihre vorzugsweise abgeflachte Schraubenspitze gegen einen von der Instrumentenaufnahme umfassten Abschnitt des Instrumentenschafts (12) presst, um somit das medizinische Instrument (10) in der Instrumentenaufnahme zu halten.

4. Lageerfassungssystem nach Anspruch 2 oder 3,
**dadurch gekennzeichnet, dass**
das Fixiermittel eine Klemmeinrichtung aufweist.

5. Lageerfassungssystem nach einem der vorangehenden Ansprüche,
**dadurch gekennzeichnet, dass**
die erste Vorrichtung eine Sensoraufnahme zur Aufnahme des Lokalisierungselements (20) aufweist.

6. Lageerfassungssystem nach einem der Ansprüche 1 bis 3,
**dadurch gekennzeichnet, dass**
das Lokalisierungselement (20) der ersten Vorrichtung an dem Haltemittel nicht lösbar angeordnet ist.

7. Lageerfassungssystem nach einem der vorangehenden Ansprüche,
**dadurch gekennzeichnet, dass**
die erste Vorrichtung eine im Wesentlichen zylinderförmige Außenfläche aufweist.

8. Lageerfassungssystem nach einem der vorangehenden Ansprüche,
**dadurch gekennzeichnet, dass**
das Haltemittel einen sich in Längsrichtung erstreckenden Kanal aufweist, wobei der Kanal zu beiden Stirnseiten des Haltemittels hin offen ist, wobei mindestens die Instrumentenspitze (14) des medizinischen Instruments (10) durch den Kanal durchführbar ist.

9. Lageerfassungssystem nach Anspruch 8,
**dadurch gekennzeichnet, dass**
der Kanal im Wesentlichen einen ovalen Querschnitt aufweist.

10. Lageerfassungssystem nach einem der vorangehenden Ansprüche,
**dadurch gekennzeichnet, dass** die weitere Vorrichtung auf dem Referenzpunkt (24) einen Drucksensor oder einen elektrischen Stromkreis, der durch den Kontakt vom Arbeitspunkt (16) der Instrumentenspitze (14) und Referenzpunkt schließbar ist, aufweist.

11. Lageerfassungssystem nach einem der vorstehenden Ansprüche,
**dadurch gekennzeichnet, dass**
der Referenzpunkt als Mulde oder als Erhebung ausgebildet ist.

12. Lageerfassungssystem nach Anspruch 11,
**dadurch gekennzeichnet, dass** die Mulde einen Mittelpunkt aufweist, wobei der Mittelpunkt der Mulde eine weitere lokale Vertiefung mit kleinerem Muldenradius aufweist.

13. Verfahren unter Verwendung eines Lageerfassungssystems nach einem der vorangehenden Ansprüche, wobei die erste Vorrichtung mit dem medizinischen Instrument (10) verbunden ist, mit dem das an dem medizinischen Instrument (10) gehaltene Lokalisierungselement (20) die ersten Vorrichtung auf den Arbeitspunkt (16) des medizinischen Instruments (10) kalibrierbar ist, wobei bei dem Verfahren mit dem Arbeitspunkt (16) der Instrumentenspitze (14) der Referenzpunkt (24) angefahren und gleichzeitig die Lage sowie Orientierung des an dem Instrument gehaltenen Lokalisierungselements (20) der ersten Vorrichtung erfasst wird, bei dem als Startbedingung für eine Kalibrierung des Lokalisierungselements der ersten Vorrichtung auf die Instrumentenspitze geprüft wird, ob sich der anzufahrende Referenzpunkt innerhalb dem Suchsektor aufgespannten Positionsdatenraums befindet.

## Claims

1. Position detection system comprising a first device with a localization element (20) for connecting a medical instrument (10) to the position detection system, a further device with a localization element and a control unit (26),
wherein the medical instrument (10) comprises an instrument shaft (12), an instrument tip (14), and a working point (16),
wherein each localization element (20) for determining location information of the respective device is arranged on the respective device within a coordinate system of the position detection system,
wherein the first device comprises at least one holding means for fastening to the medical instrument(10)
and each locating element (20) comprises a sensor coil for use in position sensing systems having an alternating electromagnetic field generated by a field generator
and each device is connectable via a respective cable (18) to the control unit (26) of the position detection system in order to transmit signals from the localization elements (20) to the control unit (26),
wherein the further device has a reference point on the locating element,
and the control unit (26) is configured to calibrate the localization element of the first device via the reference point to the working point of the medical instrument for calibration, and
to check - as a start condition for a calibration of the localization element of the first device to the instrument tip - whether the reference point is located within a position data space spanned by a search sector related to the localization element of the first device.

2. Position detection system according to claim 1, **characterized in that** the holding means comprises an instrument receptacle, wherein at least a partial section of the medical instrument can be engaged with the instrument (10) receptacle and can be fixed to the instrument receptacle via at least one fixing means of the first device.

3. Position detection system according to claim 1 or 2, **characterized in that** the fixing means comprises a screw which is screwable into the first device in such a way that as a result its preferably flattened screw tip presses against a portion of the instrument shaft (12) encompassed by the instrument (10) receptacle, thus holding the medical instrument in the instrument receptacle.

4. Position detection system according to claim 2 or 3, **characterized in that** the fixing means comprises a clamping device.

5. Position detection system according to any one of the preceding claims, **characterized in that** the first device has a sensor receptacle for receiving the localization element (20).

6. Position detection system according to one of claims 1 to 3, **characterized in that** the localization element (20) of the first device is arranged on the holding means in a non-detachable manner.

7. Position detection system according to any one of the preceding claims, **characterized in that** the first device has a substantially cylindrical outer surface.

8. Position detection system according to any one of the preceding claims, **characterized in that** the holding means comprises a longitudinally extending channel, the channel being open towards both end faces of the holding means, wherein at least the instrument tip (14) of the medical instrument (10) can be passed through the channel.

9. Position detection system according to claim 8, **characterized in that** the channel has a substantially oval cross-section.

10. A Position detection system according to any of the preceding claims, **characterized in that** the further device comprises on the reference point (24) a pressure sensor or an electric circuit closable by the contact from the working point (16) of the instrument tip (14) and reference point.

11. Device according to one of the preceding claims, **characterized in that** the reference point is formed as a depression or an elevation.

12. The Position detection system of claim 11, **characterized in that** the trough has a center point, wherein the center point of the trough has a further local depression with a smaller trough radius.

13. A method using a position detection system according to any one of the preceding claims, wherein the first device connected to the medical instrument (10), with which the localization element (20) of the first device held on the medical instrument (10) can be calibrated to the working point (16) of the medical instrument (10), in which method the reference point (24) is approached with the working point (16) of the instrument tip (14) and at the same time the position as well as the orientation of the localization element (20) of the first device held on the instrument is detected, in which, as a start condition for a calibration of the localization element of the first device to the instrument tip, it is checked whether the reference point to be approached is located within the position data space spanned by the search sector.

## Revendications

1. Système de relevé de position comprenant un premier montage ayant un élément (20) de localisation pour le rattachement d'un instrument (10) médical au système de relevé de position, un autre montage ayant un élément de localisation et une unité (26) de commande,
dans lequel l'instrument (10) médical a une tige (12) d'instrument, une pointe (14) d'instrument ainsi qu'un point (16) de travail,
dans lequel chaque élément (20) de localisation est monté sur le montage respectif pour la détermination d'informations de position du montage respectif dans un système de coordonnées du système de relevé de position, dans lequel le premier montage a au moins un moyen de retenue pour la fixation à l'instrument (10) médical
et chaque élément (20) de localisation a une bobine de capteur pour l'insertion dans des systèmes de relevé de position comprenant un champ alternatif électromagnétique produit par un générateur de champ
et chaque montage peut, par un câble (18) respectif, être relié à l'unité (26) de commande du système de relevé de position afin de transmettre des signaux des éléments (20) de localisation à l'unité (26) de commande,
dans lequel l'autre montage a un point de référence sur l'élément de localisation,
et l'unité (26) de commande est constituée pour calibrer, pour l'étalonnage, l'élément de localisation du premier montage par le point de référence sur le point de travail de l'instrument médical et
pour contrôler sur la pointe de l'instrument, comme condition initiale d'un étalonnage de l'élément de localisation du premier montage, si le point de référence se trouve dans un espace de données de position, qui est engendré par un secteur de recherche et qui est rapporté à l'élément de localisation du premier montage.

2. Système de relevé de position suivant la revendication 1, **caractérisé en ce que**
le moyen de retenue a un logement d'instrument, dans lequel au moins un tronçon partiel de l'instrument (10) médical peut être mis en prise avec le logement d'instrument et peut, par au moins un moyen de fixation du premier montage, être fixé sur le logement d'instrument.

3. Système de relevé de position suivant la revendication 1 ou 2,
**caractérisé en ce que**
le moyen de fixation comprend une vis, qui peut être vissée dans le premier montage, de manière, en résultat, à presser sa pointe de vis, de préférence aplatie, sur un tronçon, entouré du logement d'instrument, de la tige (12) de l'instrument, afin de retenir ainsi l'instrument (10) médical dans le logement d'instrument.

4. Système de relevé de position suivant la revendication 2 ou 3,
**caractérisé en ce que**
le moyen de fixation a un dispositif de serrage.

5. Système de relevé de position suivant l'une des revendications précédentes,
**caractérisé en ce que**
le premier montage a un logement de capteur pour la réception de l'élément (20) de localisation.

6. Système de relevé de position suivant l'une des revendications 1 à 3,
**caractérisé en ce que**
l'élément (20) de localisation est monté de manière inamovible sur le moyen de retenue.

7. Système de relevé de position suivant l'une des revendications précédentes,
**caractérisé en ce que**
le premier montage a une surface extérieure sensiblement de forme cylindrique.

8. Système de relevé de position suivant l'une des revendications précédentes,
**caractérisé en ce que**
le moyen de retenue a un conduit s'étendant dans la direction longitudinale, dans lequel le conduit est ouvert des deux côtés frontaux du moyen de retenue, dans lequel au moins la pointe (14) de l'instrument (10) médical peut passer dans le conduit.

9. Système de relevé de position suivant la revendication 8, **caractérisé en ce que**
le conduit a une section transversale sensiblement ovale.

10. Système de relevé de position suivant l'une des revendications précédentes,
**caractérisé en ce que**
l'autre montage a, au point (24) de référence, un capteur de pression ou un circuit électrique, qui peut être fermé par le contact du point (16) de travail de la pointe (14) de l'instrument et du point de référence.

11. Système de relevé de position suivant l'une des revendications précédentes,
**caractérisé en ce que**
le point de référence est constitué sous la forme d'une dépression ou d'une surélévation.

12. Système de relevé de position suivant la revendication 11, **caractérisé en ce que**
la dépression a un point médian, dans lequel le point médian de la dépression a une autre cavité locale ayant un rayon de dépression plus petit.

13. Procédé en utilisation un système de relevé de position suivant l'une des revendications précédentes, dans lequel le premier montage est relié à l'instrument (10) médical, par lequel l'élément (20) de localisation, retenu sur l'instrument (10) médical, du premier montage peut être étalonné sur le point (16) de travail de l'instrument (10) médical, dans lequel, dans le procédé, on met en mouvement, par le point (16) de travail de la pointe (14) de l'instrument, le point (24) de référence et, en même temps, on relève la position ainsi que l'orientation de l'élément (20) de localisation, retenu sur l'instrument, du premier montage, dans lequel, comme condition initiale d'un étalonnage de l'élément de localisation du premier montage, on contrôle, sur la pointe de l'instrument, si le point de référence mis en mouvement se trouve dans l'espace de données de position engendré dans le secteur de recherche.
